# EUROPÄISCHE PATENTSCHRIFT

(11) **EP 3 228 649 B1**
(45) Veröffentlichungstag und Bekanntmachung des Hinweises auf die Patenterteilung: **15.07.2020**
(21) Anmeldenummer: 16163614.7
(22) Anmeldetag: 04.04.2016
(51) Int. Cl.: C08G 65/30, C07C 41/36, B01J 39/20, C07C 43/11

(54) **AUFBEREITUNG ALKALISCH KATALYSIERTER ALKOXYLIERUNGSPRODUKTE**
TREATMENT OF ALKOXYLATION PRODUCTS OBTAINED BY ALKALINE CATALYSIS
TRAITEMENT DES PRODUITS D'ALKOXYLATION OBTENUS PAR CATALYSE ALCALINE

(43) Veröffentlichungstag der Anmeldung: 11.10.2017
(73) Patentinhaber: Evonik Operations GmbH, 45128 Essen (DE)
(72) Erfinder: Schubert, Frank, 47506 Neukirchen-Vluyn (DE); Knott, Wilfried, 45355 Essen (DE); Villa, Andrea, 46238 Bottrop (DE); Zellmer, Volker, 46240 Bottrop (DE); Wienhöfer, Gerrit, 23832 Chesterfield, VA (US)
(74) Vertreter: Evonik Patent Association

(56) Entgegenhaltungen:
- DE-A1- 10 024 313
- DE-T2- 68 903 216
- US-A- 3 271 462
- US-A- 5 182 025
- US-A- 5 342 541

## Beschreibung

Die vorliegende Erfindung betrifft ein Verfahren zur Aufbereitung von alkalisch katalysierten Alkoxylierungsprodukten unter Verwendung von sulfonsauren Ionenaustauschern. Alkalische Katalysatoren wie Alkalihydroxide und Alkalialkoholate sind in Alkoxylierungsreaktionen weit verbreitet. Dabei werden unter stark basischen Bedingungen Alkylenoxide wie Ethylenoxid und Propylenoxid üblicherweise an hydroxyl- oder carboxylfunktionelle Startverbindungen wie Alkohole, Phenole oder Carbonsäuren addiert. Die erhaltenen Alkoxylierungsprodukte, häufig Polyether, Polyetherole oder Polyetherpolyole genannt, enthalten im Rohzustand die Reste des alkalischen Katalysators und müssen in den meisten Fällen vor der Applikation in einem nachgeschalteten Prozessschritt aufgearbeitet, das heißt neutralisiert, von alkalischen und Salzresten befreit und filtriert werden.

Die Neutralisation geschieht häufig durch Zugabe von wässriger Phosphorsäure oder Schwefelsäure. Die Katalysatorreste werden dabei zunächst in Alkaliphosphate, Alkalihydrogenphosphate, Alkalisulfate oder Alkalihydrogensulfate umgewandelt, nach Abdestillation von Wasser gefällt und anschließend durch Filtration entfernt. Die Entfernung von Alkalisalzen ist häufig ein zeitaufwändiger und qualitätsrelevanter Schritt. In der Regel gelingt die Salzabtrennung nicht quantitativ, da ein Teil im Polyether gelöst bleibt, ein anderer Teil so feinkristallin vorliegt, dass er mit vertretbaren technischen Mitteln auch durch Zuhilfenahme von Filterhilfsmitteln nicht vollständig aus dem Endprodukt entfernt werden kann. Im Produktionsreaktor bleiben nach der Neutralisation mit Polyether behaftete Salzreste zurück, die bei Batchbetrieb vor dem Start der nächsten Produktion gelöst bzw. ausgewaschen werden müssen. Auf diese Weise wird ein mit organischen und Salzresten verunreinigtes Abwasser generiert. Als Abfallprodukt fällt ein feuchter und Polyether enthaltender Filterkuchen an, der entsorgt werden muss und zu einem Verlust an Ausbeute führt.

Die Neutralisation der alkalischen Alkoxylate mit Carbonsäuren wie Essigsäure oder Milchsäure führt je nach chemischem Aufbau des Polyethers oft zu löslichen Alkalicarboxylaten, die nicht durch Fällung und Filtration entfernt werden können. Einerseits entfallen dadurch einige der vorgenannten Aufarbeitungsschritte und Nachteile, andererseits stellen die im Endprodukt gelösten Alkalicarboxylate für viele Anwendungen unerwünschte Nebenprodukte dar. So wirken sich Carboxylatanteile störend bei Folgeumsetzungen der neutralisierten Polyether aus. In Platin-katalysierten Hydrosilylierungsreaktionen von Wasserstoffsiloxanen mit terminal ungesättigten Polyethern wie Allylpolyethern stellen sie häufig Katalysatorgifte dar, die den Pt-Katalysator inhibieren. Während die Alkalicarboxylate im Polyether gelöst sind, so fallen sie in der chemischen Weiterverarbeitung, z.B. nach der Modifikation der Polyether mit hydrophoben Struktureinheiten wie Siloxanen oder Kohlenwasserstoffresten aus dem entstehenden Reaktionsprodukt aus und verursachen inakzeptable Trübungen. Die hohe Viskosität der Folgeprodukte macht eine nachträgliche Filtration der störenden Salzreste oft unmöglich, so dass die Entfernung von Salzen direkt aus den Alkoxylierungsprodukten und vor der Weiterverarbeitung geschehen sollte.

Polyether sind sehr vielseitig eingesetzte Verbindungen. Eine wichtige Klasse von Folgeprodukten stellen Polyether-Siloxan-Copolymere, auch Polyethersiloxane, Polyethersilicone oder Siliconpolyether genannt, dar. Die Anwendungsbreite beruht auf der Möglichkeit, durch geeignete Kombination von Siloxan- und Polyetherstruktur vielfältige Wirkprinzipien gezielt einzustellen. Von besonderer Bedeutung sind dabei Polyether, die sich von Allylalkohol ableiten und in Gegenwart von Pt-Katalysatoren mit Si-H funktionellen Siloxanen zu SiC-verknüpften Polyethersiloxanen umgesetzt werden. Während der alkalisch katalysierten Herstellung von Allylpolyethern kommt es unvermeidbar zur Isomerisierung eines Teils der Allylgruppen zu thermodynamisch stabileren Propenylgruppen. Die Hydrosilylierungsreaktion erfordert für die Si-C-Verknüpfungsreaktion terminale Doppelbindungen, so dass die entstandenen Propenylpolyether im Sinne der Polyethersiloxansynthese unreaktive Nebenprodukte darstellen. Dem wird Rechnung getragen, indem zur Sicherung eines quantitativen Si-H-Umsatzes ein beträchtlicher Überschuss der Polyetherkomponente in der Hydrosilylierung eingesetzt wird.

Wie die DE 10024313 A1 ausweist, ist die Präsenz von Propenylpolyethern Ursache für verschiedene weitere unerwünschte Eigenschaften. Unter dem Einfluss von (Luft)Feuchtigkeit und begünstigt durch Säurespuren unterliegen Propenylpolyether der Hydrolyse. Propionaldehyd wird im Laufe der Zeit freigesetzt und gast teilweise aus. In Sekundärreaktionen entstehen aus Propionaldehyd cyclische Oligomere (Aldoxane, Trioxane) sowie Acetale, die die Tendenz zur Rückspaltung und damit zur erneuten Aldehydfreisetzung aufweisen. Vor allem Produkte, die im Bereich Personal Care und in Innenräumen eingesetzt werden, erfordern eine geruchliche Neutralität und somit häufig eine Nachbehandlung. Acetale entstehen oftmals schon während der Polyetherherstellung durch Reaktion von Aldehyd mit dem OH-funktionellen Polyether. Acetale erhöhen die Viskosität durch Molmassenaufbau und verzerren die angestrebten Eigenschaften der Endprodukte.

Der Stand der Technik beschreibt verschiedene Methoden zur Vermeidung oder Behebung der dargestellten Problematik bei Allylpolyether basierenden Systemen:
Die EP 0118824 A1 beschreibt Polyethersiloxane als Öle für kosmetische Zwecke mit einem Gesamtgehalt an Carbonylgruppen tragenden Verbindungen (Aldehyde und Ketone) von ≤100 ppm, die durch Hydrosilylierung in Gegenwart von Antioxidantien und ggfs. eines Puffers gewonnen werden.

Die JPH 07304627 A offenbart ein Verfahren zur Behandlung Allylpolyether basierender Polyethersiloxane mit wässriger Salzsäure bei 60 °C innerhalb von 24 h. Eine Säure-induzierte Hydrolyse von Propenylpolyetheranteilen unter Entfernung von Propionaldehyd ist auch in J. Soc. Cosmet. Japan (1993), 27(3), 297-303 beschrieben. Die EP 0398684 A2 beschreibt die Herstellung geruchsarmer Siliconpolyether durch die Behandlung mit verdünnter Salzsäure für einige Stunden in der Wärme mit anschließender Vakuumdestillation, wobei ein nahezu geruchloses Copolymer gewonnen wird.

Der Zusatz von Phytinsäure führt nach den Ausführungen der US 4,515,979 ebenfalls zu einer Minderung unerwünschter Gerüche in Polyethersiloxanen auf Basis von Allylpolyethern. Nachteilig ist, dass die Phytinsäure im Endprodukt verbleibt und die Anwendung in sensiblen Bereichen wie in Lacken und Personal Care Produkten verhindert. Verfahren wie die katalytische Druckhydrierung sind aufwendig und teuer und somit nur für kleine, hochpreisige Anwendungsfelder vertretbar.

Wie in der EP 1531331 A2 dargelegt, sind die nach den bekannten Verfahren mit Säure behandelten Polyethersiloxane für den Einsatz als Polyurethanschaumstabilisatoren ungeeignet. Die Säurebehandlung hat desaströse Auswirkungen auf die Anwendungseigenschaften und an Stelle der gewünschten Schaumstabilisierung wird besonders in Weichschaumsystemen ein Kollaps der labilen Schaumstruktur beobachtet. Stattdessen wird eine schonende Behandlung der Siliconpolyether mit Wasserstoffperoxid gefolgt von einer destillativen Abtrennung geruchsbildender Beimengungen bevorzugt.

Der Stand der Technik kennt alternative Alkoxylierungskatalysatoren, die den Zugang zu salzfreien und nahezu propenylfreien und olfaktorisch günstigen Polyethern gewähren. Dazu gehören Doppelmetallcyanid- (DMC)-Katalysatoren, wie z.B. in der EP 2241352 A2 ausgeführt. Wie dem Fachmann bekannt, führen DMC-Katalysatoren auf Grund ihres gänzlich anderen Wirkmechanismus zu Polyethern mit sehr enger Molmassenverteilung. Die Sequenz von Ethylenoxy- und Propylenoxy-Einheiten bei gemischten Polyethern unterscheidet sich in statistisch gemischten Alkoxylaten von derjenigen in alkalisch katalysierten Polyethern. Beides hat Einfluss auf die Produkteigenschaften wie die Hydrophilie/Hydrophobie, den Trübungspunkt oder die Kompatibilität in verschiedenen Medien. Ferner unterliegt der Einsatz der DMC-Katalyse gewissen Einschränkungen. Vor allem die für Polyethersiloxane wichtigen Allyl- und Butyl-Polyether lassen sich mit Hilfe von DMC-Katalysen nicht auf dem direkten Wege herstellen, da z.B. kurzkettige Alkohole den DMC-Katalysator inhibieren. Die DMC-Katalyse stellt somit für viele Anwendungen keine funktionierende Alternative zur weit verbreiteten alkalischen Katalyse dar.

In der DE 10024313 A1 wird ein Verfahren dargelegt, in dem ein Kationenaustauscher eingesetzt wird, um Alkalimetallionen aus alkalischen Alkoxylaten zu entfernen und einen Eintrag von Phosphat ins Endprodukt zu vermeiden. Das alkalische Alkoxylierungsprodukt wird dabei in einem inerten organischen Lösemittel gelöst, bei 20-60 °C mit einem Kationenaustauscher behandelt und zum Schluss vom Lösemittel befreit.

Die US 5,342,541 offenbart die Verwendung saurer Kationenaustauscher mit dem Ziel, den Gehalt an Propenylpolyethern im Endprodukt zu reduzieren. Nachteilig an dem Verfahren ist der Eintrag von Säurespuren aus den verwendeten Gel-Typ-Ionenaustauschern in die damit behandelten Polyether, welche den direkten Einsatz der Produkte in Polyurethanen praktisch unmöglich machen. Das Verfahren erfordert daher eine Nachbehandlung der aziden Polyether mit einer Epoxidverbindung als Säurefänger. Die Anwendbarkeit des Prozesses beschränkt sich auf gelartige Ionenaustauscher, da nur diese über so kleine Poren verfügen, dass langkettige Polymere keinen Zugang haben. Durch den vermiedenen direkten Kontakt mit den sauren Sulfonsäuregruppen wird eine Degradation des Polyethers unterdrückt.

Somit besteht die Aufgabe der vorliegenden Erfindung in der Bereitstellung eines schonenden, umweltfreundlichen und effizienten Verfahrens zur Aufreinigung von alkalisch katalysierten Alkoxylierungsprodukten sowie in der Bereitstellung entsprechend aufgereinigter Alkoxylierungsprodukte.

Überraschend wurde gefunden, dass qualitativ hochwertige und vielseitig einsetzbare aufgereinigte Polyether erhalten werden, wenn die alkalisch katalysierten Rohprodukte in alkoholisch-wässrigen Lösungen bei erhöhten Temperaturen von mehr als 40 °C mit sulfonsauren Ionenaustauschern, vorzugsweise mit speziell ausgewählten makroporösen sulfonsauren Ionenaustauschern, behandelt werden.

Gegenstand der vorliegenden Erfindung ist daher ein Verfahren zur Aufbereitung von alkalisch katalysierten Alkoxylierungsprodukten unter Verwendung von sulfonsauren Ionenaustauschern, umfassend
a) Bereitstellen einer Mischung, umfassend das aufzubereitende alkalisch katalysierte Alkoxylierungsprodukt, Alkohol mit 1 bis 4 Kohlenstoffatomen und Wasser,
b) Behandeln der aus Schritt a) erhaltenen Mischung mit einem sulfonsauren Kationenaustauscher bei >40 °C,
c) Abtrennung, vorzugsweise destillative Abtrennung, des Alkoxylierungsproduktes aus der in Schritt b) erhaltenen Mischung,
wobei die aufzubereitenden alkalisch katalysierten Alkoxylierungsprodukte Allyl-Polyether und darin enthaltene Propenylpolyether umfassen, zur Entfernung von Alkalimetallresten und geruchsbildenden Beimengen aus den alkalisch katalysierten Alkoxylierungsprodukten, wobei bei der Aufbereitung des alkalisch katalysierten Alkoxylierungsproduktes eine Reduzierung der Propenylgruppen erfolgt.

Die Begriffe "Alkoxylat" und "Alkoxylierungsprodukt" werden im Sinne dieser Erfindung synonym verwendet und umfassen insbesondere die durch alkalisch katalysierte Polyaddition von Alkylenoxiden an Hydroxylgruppen und/oder Carboxylgruppen entstandenen Reaktionsprodukte, auch bekannt als Polyether, Polyole, Polyetherole, Polyethylenglykol oder Polypropylenglykole. Dies schließt Reinsubstanzen wie auch Gemische, die unter Verwendung verschiedener Alkylenoxide und/oder verschiedener Hydroxylgruppen und/oder Carboxylgruppen tragender Startverbindungen mit ein.

Durch den Gegenstand der Erfindung wird es nicht nur ermöglicht, Alkalimetallionen bzw. Alkalimetalle aus den alkalischen Alkoxylaten zu entfernen und die Alkoxylate zu neutralisieren, sondern darüber hinaus ungewünschte geruchsbildende Verbindungen oder Beimengungen wie Propenylpolyether oder Acetale zu beseitigen und somit den Zugang zu praktisch salzfreien und olfaktorisch günstigen, vielseitig verwendbaren Polyethern zu gewährleisten.

Die Kombination aus Lösemittelgemisch, umfassend Alkohol und Wasser, sowie vorzugsweise kurzen Kontaktzeiten in der Hitze (T>40°C) an sulfonsauren Ionenaustauschern, vorzugsweise mit dezidiert ausgewählten Porengrößen, ermöglicht die Entsalzung und die Eliminierung von geruchsbildenden Inhaltsstoffen in nur einem einzigen Verfahren.

Es wird ermöglicht, aufgereinigte und praktisch salzfreie Alkoxylierungsprodukte mit allenfalls geringem Rest-Säuregehalt zur Verfügung zu stellen.

Es wird ermöglicht, aufgereinigte Polyether bereit zu stellen, die einen verringerten Gehalt an geruchsbildenden Beimengungen, wie z.B. an Propenylethern, Aldehyden und Acetalen, aufweisen, so dass sie keiner weiteren Nachbehandlung bedürfen und direkt zur Herstellung von Folgeprodukten eingesetzt werden können.

Die erfindungsgemäß hergestellten aufgereinigten Polyether kombinieren die für manche Applikationen bedeutsame und für alkalisch katalysierte Polyether typische breitere Molmassenverteilung mit den Vorzügen der grundsätzlich Salz- und Propenyl-freien DMC-katalysierten Polyether.

In der Folge ermöglicht die vorliegende Erfindung die Verwendung der erfindungsgemäß erhältlichen Alkoxylierungsprodukte bei der Herstellung von PUR-Schaum, Polymeren wie Polyethersiloxanen und Polyestern, als Polyurethanschaumstabilisatoren, in Lacken, Beschichtungen, Kleb- und Dichtstoffen, Bindemitteln, kosmetischen Zubereitungen, Körperpflegemitteln sowie Reinigungsmitteln, als Tenside, Emulgatoren, Dispergiermittel, Entschäumer, Netzmittel, Reibminderer, Schmiermittel, Gleitmittel, Trennmittel, Additive in Kraftstoffen wie Benzin und Diesel und Rheologiemodifizierer und die Bereitstellung besonders hochwertiger Folgeprodukte, die sich z.B. durch besondere Geruchsneutralität auszeichnen.

Die Begriffe "alkalimetallfrei" und "salzfrei" bedeuten im Sinne dieser Erfindung, dass vorzugsweise weniger als 10 ppm, insbesondere weniger als 5 ppm Alkalimetalle enthalten sind.

Das erfindungsgemäße Verfahren dient der Entfernung von Alkalimetallresten und geruchsbildenden Beimengen aus den alkalisch katalysierten Alkoxylierungsprodukten.

Zur bevorzugten Ausgestaltung von Schritt a) des erfindungsgemäßen Verfahrens:
Die in Schritt a) eingesetzten Alkoxylierungsprodukte sind alkalisch katalysierte Alkoxylierungsprodukte. Solche sind dem Fachmann an sich wohlbekannt. Sie können nach den im Stand der Technik bekannten Methoden in Gegenwart von Alkalihydroxid- oder Alkalialkoholat-Katalysatoren hergestellt werden und enthalten gewöhnlich 100 ppm bis 6000 ppm, bevorzugt 500 ppm bis 4000 ppm Alkalimetalle.

Weit verbreitet sind z.B. alkalisch katalysierte Alkoxylate, die unter Einsatz von Natriumhydroxid, Kaliumhydroxid, Natriummethanolat und/oder Kaliummethanolat synthetisiert wurden. Solche alkalisch katalysierte Alkoxylate können im Sinne der vorliegenden Erfindung bevorzugt eingesetzt werden.

Das erfindungsgemäße Verfahren ist auf alkalische Alkoxylierungsprodukte beliebiger Molmasse anwendbar. Bevorzugt sind Alkoxylierungsprodukte mit gewichtsmittleren Molmassen Mw von 150 g/mol bis 15 000 g/mol, vorzugsweise 200 g/mol bis 10 000 g/mol, besonders bevorzugt 400 g/mol bis 5 000 g/mol. Die gewichtsmittleren Molmassen Mw sind bestimmbar über GPC-Methode: Säulenkombination SDV 1000/10000 Å (Länge 65 cm), Temperatur 30 °C, THF als mobile Phase, Fließrate 1 ml/min, Probenkonzentration 10 g/l, RI-Detektor, Auswertung gegen Polypropylenglykol-Standard.

Die Polydispersität der eingesetzten Alkoxylierungsprodukte ist in weiten Bereichen variabel. Bevorzugt eingesetzte alkalische Alkoxylate weisen nach GPC-Methode gegen PPG-Standard eine Polydispersität von Mw/Mn von 1,04 bis 1,5, besonders bevorzugt zwischen 1,05 und 1,35 auf.

Es entspricht einer besonders bevorzugten Ausführungsform der Erfindung, wenn die aufzubereitenden alkalisch katalysierten Alkoxylierungsprodukte aus einem mit Alkalihydroxid und/oder Alkalialkoholat katalysierten Alkoxylierungsprozess stammen, eine Molmasse Mw (GPC-Methode gegen PPG-Standard) von 150 g/mol bis 15 000 g/mol, vorzugsweise 200 g/mol bis 10 000 g/mol, besonders bevorzugt 400 g/mol bis 5 000 g/mol und eine Polydispersität von 1,04 bis 1,5, besonders bevorzugt zwischen 1,05 und 1,35 aufweisen.

Sowohl bei Raumtemperatur (20°C) flüssige als auch bei Raumtemperatur feste Alkoxylierungsprodukte sind einsetzbar, da diese vor der Ionenaustauscherbehandlung in ein Lösemittelgemisch gegeben werden. Die Viskosität der resultierenden Mischung kann durch die Lösemittelmenge eingestellt werden.

Die in Schritt a) eingesetzten alkalisch katalysierten Alkoxylate sind insbesondere die Reaktionsprodukte einer Polyaddition von Epoxidverbindungen an eine OH-funktionelle oder Carboxyl-funktionelle Startverbindung. Als Alkylenoxide werden bevorzugt Ethylenoxid, Propylenoxid, 1-Butylenoxid, 2-Butylenoxid, Isobutylenoxid und Styroloxid, besonders bevorzugt Ethylenoxid und Propylenoxid eingesetzt. Die Epoxidmonomere können in reiner Form, nacheinander oder gemischt eingesetzt werden. Die entstehenden Polyoxyalkylene unterliegen damit einer statistischen Verteilung im Endprodukt. Die Zusammenhänge zwischen Dosierung und Produktstruktur sind dem Fachmann bekannt.

Als OH-funktionelle Starter eignen sich grundsätzlich alle gesättigten oder ungesättigten, linearen oder verzweigten ein- oder mehrfach OH-funktionellen Startverbindungen. Bevorzugte Starter sind Verbindungen aus der Gruppe der Alkohole, Diole, Polyole, Polyetherole und Phenole, bevorzugt Allylalkohol, n-Butanol, 1-Octanol, 1-Decanol, 1-Dodecanol, generell Fettalkohole mit 8-22 Kohlenstoffatomen wie Stearylalkohol, 2-Ethylhexanol, Isononanol, 3,5,5-Trimethylhexanol, Cyclohexanol, Benzylalkohol, 1,2-Hexandiol, 1,6-Hexandiol, 1,4-Butandiol, Neopentylglykol, Hexylenglykol, Eugenol, Alkylphenole, Cashew Nut Shell Liquid, Hexenol, Ethylenglykol, Propylenglykol, Di-, Tri- und Polyethylenglykol, 1,2-Propylenglykol, Di- und Polypropylenglykol, Trimethylolpropan, Glycerin, Polyglycerin, Pentaerythrit, Sorbit oder sich von Naturstoffen ableitende, Hydroxylgruppen tragende Verbindungen.

Bevorzugte Startverbindungen weisen pro Molekül im Mittel 1 bis 6, vorzugsweise 1 bis 3, besonders bevorzugt 1 bis 2, ganz besonders bevorzugt 1 OH-Gruppe auf.

Wenn die aufzubereitenden alkalisch katalysierten Alkoxylierungsprodukte folglich 1 bis 6 OH-Gruppen, bevorzugt 1 bis 3 OH-Gruppen, besonders bevorzugt 1 bis 2 OH-Gruppen, insbesondere 1 OH Gruppe aufweisen, so liegt eine bevorzugte Ausführungsform der Erfindung vor.

Darüber hinaus können auch beliebige Carbonsäuren als Starter verwendet werden. Bevorzugt sind ein- oder mehrwertige aliphatische Carbonsäuren, aromatische Carbonsäuren und cycloaliphatische Carbonsäuren. Insbesondere bevorzugt sind aliphatische, gesättigte oder ungesättigte, lineare oder verzweigte Carbonsäuren mit 6 bis 22 Kohlenstoffatomen wie z.B. Decansäure, Undecansäure, Dodecansäure, Octadecansäure, 2-Ethylhexancarbonsäure, Isononansäure, 3,5,5-Trimethylhexancarbonsäure, Neodecansäure, Isotridecancarbonsäure, Isostearinsäure, Undecylensäure, Ölsäure, Linolsäure und Ricinolsäure. Ebenfalls bevorzugt sind aromatische Carbonsäuren wie Benzoesäure und Zimtsäure.

Ganz besonders bevorzugt eingesetzt werden Allyl-Polyether, da bei diesen Produkten der Nutzen des erfindungsgemäßen Verfahrens in Form eines weitgehenden Abbaus von darin enthaltenen Propenylpolyethern ganz besonders ausgeprägt ist.

Das alkalische Alkoxylierungsprodukt wird erfindungsgemäß mit Alkohol mit 1 bis 4 Kohlenstoffatomen und Wasser gemischt. Als Alkohole eignen sich Methanol, Ethanol, 1-Propanol, Isopropanol, 1-Butanol, 2-Butanol und Isobutanol, wobei Methanol und Ethanol bevorzugt verwendet werden. Als weitere Lösemittelkomponente wird Wasser zugesetzt. Das Verhältnis von Alkohol zu Wasser ist in weiten Grenzen variabel und wird der Polyetherstruktur und damit der Löslichkeit des jeweils aufzureinigenden Alkoxylierungsprodukts angepasst. Das Verhältnis von Alkoxylierungsprodukt zu Alkohol und Wasser wird nach erfindungsgemäßem Verfahren vorzugsweise so gewählt, dass eine homogene, möglichst klare Lösung entsteht.

Zur Erhöhung der Wirtschaftlichkeit und zur Vermeidung von Abfällen (Recycling) kann das in Schritt c) zurückgewonnene Alkohol/Wasser-Destillat zur Herstellung der Alkoxylatlösung in Schritt a) wieder genutzt werden. Bei Bedarf kann diesem Destillat reines alkoholisches Lösemittel und/oder Wasser zugemischt werden, um die benötigte Lösungszusammensetzung einzustellen.

Die mit dem Ionenaustauscher zu behandelnde Mischung, insbesondere Lösung, besteht vorteilhafterweise zu 35 bis 95 Gew.-%, bevorzugt zu 45 bis 85 Gew.-%, besonders bevorzugt zu 50 bis 80 Gew.-% aus dem alkalischen Alkoxylierungsprodukt. In der Mischung, vorzugsweise Lösung, beträgt der Anteil des Alkohols vorteilhafterweise 4 bis 64 Gew.-%, bevorzugt 12 bis 53 Gew.-%, besonders bevorzugt 17 bis 48 Gew.-%. Der Wassergehalt der Lösung beträgt vorzugsweise 1 bis 15 Gew.-%, bevorzugt 2 bis 10 Gew.-%, besonders bevorzugt 3 bis 8 Gew.-%. Die Summe aus Alkoxylierungsprodukt, Alkohol und Wasser ergänzt sich zu 100 Gew.-%, sofern keine weiteren Stoffe enthalten sind.

Zur bevorzugten Ausgestaltung von Schritt b) des erfindungsgemäßen Verfahrens:
Im Sinne des erfindungsgemäßen Verfahrens können grundsätzlich alle bekannten sulfonsauren Kationenaustauscher eingesetzt werden. Kationenaustauscher mit Sulfonsäuregruppen auf Kunstharzbasis, zum Beispiel sulfonierte Styrol-Divinylbenzol-Polymerisate, erweisen sich in der erfindungsgemäßen Polyetheraufreinigung als besonders wirksam. Es wurde überraschend gefunden, dass makroporöse sulfonsaure Kationenaustauscher in besonderem Maße für die Zwecke der vorliegenden Erfindung geeignet sind. Bereits kurze Kontaktzeiten von vorzugsweise weniger als 50 min, unter bevorzugten Bedingungen weniger als 40 min reichen aus, um Alkaliionen zu entfernen und z.B. die Hydrolyse von Propenylpolyethern und Acetalen zu bewirken.

Im Markt sind zahlreiche sulfonsaure Kationenaustauscher kommerziell erhältlich. Hierzu gehören Ionenaustauscher der Firmen DOW (Handelsnamen z.B. Amberlyst®, Amberjet® und Amberlite®), Lanxess (Handelsname Lewatit®) und Purolite (Purolite®).

Für das erfindungsgemäße Verfahren besonders geeignet sind gekörnte makroporöse Ionenaustauscher mit Sulfonsäuregruppen. Bevorzugte Ionenaustauscher weisen vorteilhafterweise Partikel mit einer mittleren Partikelgröße von 500-900 µm, gemessen durch Siebanalyse, und eine Ionenaustauschkapazität von größer oder gleich 1,5 equ./Liter (H⁺ Form) auf, was einer bevorzugten Ausführungsform der Erfindung entspricht. Hierzu gehören z.B. die Kationenaustauscher Amberlyst® 15 und Amberlite® 252H.

Sofort anwendbar sind z.B. jene makroporösen und wasserhaltigen sulfonierten Ionenaustauscher, die bereits ab Werk in H-Form vorliegen. Diese können ohne Vorbehandlung angewendet werden. Nach dem Gebrauch lassen sich die mit Alkaliionen ganz oder teilweise belegten bevorzugten Ionenaustauscher auf bekanntem Wege mit starken wässrigen Säuren wie Schwefelsäure oder Salzsäure regenerieren, d.h. in die H-Form zurück verwandeln und viele Male wiederverwenden.

Die Behandlung der Alkoxylatlösung aus Schritt a) mit den zuvor genannten sulfonsauren Kationenaustauschern kann sowohl im Batchverfahren als auch kontinuierlich und sowohl in Rührreaktor wie im Festbettverfahren erfolgen.

Beim Batchbetrieb in einem rührbaren Behälter können die alkoholisch-wässrige Alkoxylatlösung aus Schritt a) und der sulfonsaure Ionenaustauscher in H-Form vorgelegt und auf die gewünschte Temperatur gebracht werden. Die eingesetzte Menge an Ionenaustauscher richtet sich nach dem Alkalimetallgehalt der Alkoxylatlösung und der verfügbaren Kapazität (Gehalt an nutzbaren SO₃H-Gruppen) im Ionenaustauscher. Zur quantitativen Entfernung von Alkalimetallen aus dem Alkoxylat muss eine mindestens stöchiometrische Menge an Sulfonsäuregruppen des Ionenaustauschers bezogen auf die zu entfernenden Alkalimetallionen eingesetzt werden. Vorteilhaft ist die Verwendung einer solchen Menge an Ionenaustauscher, die einem mindestens 0,1-molaren Überschuss an Säuregruppen bezogen auf die zu entfernende Alkalikonzentration entspricht. Ein höherer lonenaustauscherüberschuss ist nicht schädlich, sondern eher förderlich für eine schnelle und gründliche Aufreinigung der Alkoxylierungsprodukte. Das Fortschreiten der Aufarbeitung wird am einfachsten durch eine eintauchende pH-Messsonde verfolgt. Die Mischung aus Ionenaustauscher und Alkoxylatlösung wird insbesondere so lange gerührt, bis der pH-Wert von anfangs 12 bis 14 auf kleiner oder gleich pH 7 gefallen ist.

Die so gewonnene alkalimetallfreie Lösung hat vorteilhafterweise einen Restgehalt an Alkalimetall bezogen auf das aufgereinigte Alkoxylierungsprodukt von kleiner als 10 ppm, bevorzugt von kleiner als 5 ppm.

Die Temperatur hat Einfluss auf die Dauer der Neutralisation und auf die zeitlich parallel stattfindende Hydrolyse von evtl. vorhandenen Propenylpolyethern und Acetalen und ist größer als 40 °C, bevorzugt größer als 60 °C, besonders bevorzugt größer als 70 °C. Die maximale Temperatur ist in offenen Systemen nur durch den Siedepunkt des Alkoxylat-Alkohol-Wasser-Gemischs begrenzt, und das erfindungsgemäße Verfahren kann auch am Siedepunkt und unter Rückfluss stattfinden. In einem geschlossenen druckfesten Rührreaktor kann die Behandlung der Alkoxylatlösung mit dem Ionenaustauscher auch oberhalb der Siedetemperatur unter Druck zum Beispiel in Ethanol/Wasser bei 100 °C ausgeführt werden.

Es entspricht einer bevorzugten Ausführungsform der Erfindung, wenn in Schritt b) die Mischung aus Schritt a) bei 45 °C bis 100 °C, bevorzugt größer als 60 °C bis 100°C, besonders bevorzugt größer als 70 °C bis 100°C durch ein Ionenaustauscherbett geleitet wird.

Die Temperatur, die Art und Menge an alkoholischem Lösemittel, der Wassergehalt, der Alkaligehalt und der chemische Aufbau des aufzureinigenden Alkoxylierungsprodukts sowie die Einsatzmenge des Ionenaustauschers beeinflussen die Dauer der Aufreinigung. Die Dauer der Aufarbeitung ist definiert als die Zeit, gemessen vom Zeitpunkt der Zugabe des Ionenaustauschers zur alkalischen Alkoxylatlösung bis zum Erreichen eines pH-Werts von 7. In einer bevorzugten Ausführungsform werden die Einflussgrößen so gewählt, dass der pH-Wert von 7 innerhalb von weniger als 50 Minuten erreicht wird. Besonders bevorzugt sind kurze Verweilzeiten von weniger als 40 Minuten und insbesondere von weniger als 25 Minuten bis zum Erreichen ein pH-Werts von 7.

Der Einsatz von mehr Lösemittel, mehr Ionenaustauscher und höherer Temperatur bewirkt in der Regel eine Beschleunigung des Ionenaustauschs und der Aufreinigung.

Das Durchleiten der alkalischen Lösung aus Schritt a) durch ein mit sulfonsaurem Ionenaustauscher gefülltes Behältnis bei >40 °C stellt eine vorteilhafte und leicht zu praktizierende Alternative zum Rührreaktorverfahren dar. Die Alkalimetalle enthaltende Alkoxylat-Alkohol-Wasser-Mischung, vorzugsweise Lösung, wird dabei z.B. mit Hilfe einer Pumpe kontinuierlich durch das temperierbare lonenaustauscherfestbett geleitet. Vorzugsweise befindet sich das lonenaustauscherfestbett in einer Säule oder in einem Rohr, welche bzw. welches von außen temperiert werden kann. So eignen sich beispielsweise mit einem Doppelmantel ausgestattete Behältnisse besonders gut, bei denen ein flüssiger Wärmeträger im Außenmantel zirkulieren kann. Angeschlossen an eine externe, steuerbare Wärmeträgeranlage und ausgestattet mit einer Temperaturmessstelle im Innern des Behältnisses, lässt sich die Temperatur im Festbett auf einen vorgegebenen Wert einstellen und über den gesamten Betriebszeitraum konstant halten.

Nach dem Durchgang durch die lonenaustauschersäule wird die aufgearbeitete Polyetherlösung in einem geeigneten Produktbehälter aufgefangen. Es ist empfehlenswert, den pH-Wert der abfließenden Lösung fortlaufend zu überwachen, um rechtzeitig zu erkennen, wenn die lonenaustauscherkapazität erschöpft ist. Die Betriebsbedingungen werden vorzugsweise so eingestellt, dass der abfließende Produktstrom einen pH-Wert von kleiner oder gleich 7 hat.

Die so gewonnene alkalimetallfreie Lösung hat vorteilhafterweise einen Restgehalt an Alkalimetall bezogen auf das aufgereinigte Alkoxylierungsprodukt von kleiner als 10 ppm, bevorzugt von kleiner als 5 ppm.

Neben der Temperatur, der Art und Menge an alkoholischem Lösemittel, dem Wasser- und Alkaligehalt sowie dem chemischen Aufbau und der Einsatzmenge des Ionenaustauschers beeinflusst beim Festbettverfahren die Einspeisegeschwindigkeit die Qualität der Aufreinigung. Die Einspeiserate bestimmt die mittlere Verweilzeit der Lösung im Festbett. In einer bevorzugten erfindungsgemäßen Ausführungsform werden die Prozessparameter so aufeinander abgestimmt, dass der pH-Wert des austretenden Produktstroms kleiner oder gleich 7 ist. Vorzugsweise wird die Einspeisegeschwindigkeit gedrosselt oder der Ionenaustauscher kann mit Säure regeneriert werden, wenn der pH-Wert des Produktstroms größer als pH 7 ist.

Bei anstehendem Produktwechsel und vor einer Regenerierung mit Säure ist es bevorzugt, das lonenaustauscherfestbett durch Spülen mit Lösemittel und/oder Wasser von Produktanhaftungen zu befreien. Von Vorteil ist, zum Auswaschen von Polyetherresten das in Schritt a) verwendete Alkohol-Wasser-Gemisch zu nutzen.

Nach der Regenerierung mit Säure ist es erforderlich, Säurereste mit Wasser und/oder einem organischen Lösemittel wie Alkohol auszuspülen. Mit Hilfe einer pH-Messsonde am Reaktorauslass kann der Endpunkt des Spülvorgangs leicht detektiert werden.

Die Einspeisung der Säure während des Regenerierungsprozesses kann entweder in der gleichen Strömungsrichtung wie beim Zuspeisen der alkalischen Alkoxylatlösung (Gleichstromverfahren) oder in entgegengesetzter Richtung (Gegenstromverfahren) geschehen. Bevorzugt ist das Gegenstromverfahren.

Zur bevorzugten Ausgestaltung von Schritt c) des erfindungsgemäßen Verfahrens:
Die aus Schritt b) resultierende Mischung wird im Schritt c) des erfindungsgemäßen Verfahrens vom Lösemittelgemisch befreit. Dies geschieht vorzugsweise durch Abdestillieren, insbesondere durch Vakuumdestillation. Bei Bedarf können letzte Lösemittelreste durch Strippen mit Wasser oder einem Inertgas wie Stickstoff aus dem Polyether entfernt werden. Die Abtrennung des Lösemittelgemischs kann sowohl batchweise als auch kontinuierlich und sowohl im Rührkessel als z.B. im Dünnschichtverdampfer durchgeführt werden.

Es ist besonders vorteilhaft, den ersten Teil des Lösemittelgemischs unter Normaldruck und den Rest schließlich unter Vakuum abzudestillieren. Gegen Ende wird die Temperatur vorzugsweise auf über 100 °C erhöht und der Druck vorzugsweise auf unter 50 mbar gesenkt bis kein Destillat mehr fließt. Das Alkohol/Wasser-Destillat kann zur Herstellung einer Lösung gemäß Schritt a) zu einem späteren Zeitpunkt wiederverwendet werden.

Das nach der Lösemittelabtrennung gewonnene aufgereinigte Alkoxylierungsprodukt ist frei von Salzen und muss in der Regel nicht filtriert werden. Eine Filtration kann dennoch optional durchgeführt werden, um etwaige Feinanteile des Ionenaustauschers zu entfernen.

Es entspricht einer bevorzugten Ausführungsform der Erfindung, wenn bei der Aufbereitung des alkalisch katalysierten Alkoxylierungsproduktes eine Reduzierung der Propenylgruppen erfolgt, wobei vorzugsweise ein um mehr als 40 %, bevorzugt 50 % bis 95 % geringerer Gehalt an Propenylgruppen resultiert, verglichen mit dem zur Aufbereitung eingesetzten Alkoxylierungsprodukt.

Ein Alkoxylierungsprodukt, erhältlich nach dem erfindungsgemäßen Verfahren wie zuvor beschrieben, wird offenbart Auf die vorgenannten bevorzugten Ausführungsformen sei verwiesen.

Das erfindungsgemäß erhältliche Alkoxylierungsprodukt weist im Rahmen einer bevorzugten Ausführungsform der Erfindung eine Säurezahl zwischen 0 und 0,5 mg KOH/g, bevorzugt kleiner oder gleich 0,3 mg KOH/g auf.

Gemäß einer weiteren bevorzugten Ausführungsform der Erfindung ist das erfindungsgemäß erhältliche Alkoxylierungsprodukt phosphatfrei und der Gehalt an Alkalimetall, vorzugsweise Natrium und Kalium, ist kleiner als 10 ppm, bevorzugt kleiner als 5 ppm.

Die erfindungsgemäß erhältlichen Produkte sind hervorragend zur Herstellung von Polyurethanschaum, von Polymeren wie Polyethersiloxanen und Polyestern, als Polyurethanschaumstabilisatoren, zur Anwendung in Lacken und zur Oberflächenbehandlung, in Beschichtungen, Kleb- und Dichtstoffen, Bindemitteln, kosmetischen Zubereitungen, Körperpflegemitteln sowie Reinigungsmitteln, als Tenside, Emulgatoren, Dispergiermittel, Entschäumer, Netzmittel, Reibminderer, Schmiermittel, Gleitmittel, Trennmittel, Additive in Kraftstoffen wie Benzin und Diesel und Rheologiemodifizierer geeignet. Allylpolyether im Speziellen zeigen in Platin-katalysierten Hydrosilylierungsreaktionen eine ausgezeichnete Reaktivität in der Umsetzung mit Wasserstoffsiloxanen selbst bei so geringen Pt-Einsatzkonzentration wie 2 ppm Pt bezogen auf den Reaktionsansatz.

Offenbart wird die Verwendung der erfindungsgemäß erhältlichen Alkoxylierungsprodukte zur Herstellung von Polymeren wie Polyethersiloxanen und Polyester, als Polyurethanschaumstabilisatoren, in Lacken und zur Oberflächenbehandlung, in Beschichtungen, Kleb- und Dichtstoffen, Bindemitteln, kosmetischen Zubereitungen, Körperpflegemitteln sowie Reinigungsmitteln, als Tenside, Emulgatoren, Dispergiermittel, Entschäumer, Netzmittel, Reibminderer, Schmiermittel, Gleitmittel, Trennmittel, Additive in Kraftstoffen wie Benzin und Diesel und Rheologiemodifizierer.

Offenbart wird ein PUR-Schaum, erhältlich durch Umsetzung mindestens einer Polyolkomponente und mindestens einer Isocyanatkomponente in Gegenwart eines Polyethersiloxans, welches unter Einsatz des erfindungsgemäß erhältlichen Alkoxylierungsproduktes erhalten wurde.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll. Das Verfahren sowie die erfindungsgemäße Verwendung werden nachfolgend beispielhaft beschrieben, ohne dass die Erfindung auf diese beispielhaften Ausführungsformen beschränkt sein soll.

### Beispiele:

### GPC-Messungen:

GPC-Messungen zur Bestimmung der Polydispersität und mittleren Molmassen Mw wurden unter den folgenden Messbedingungen durchgeführt: Säulenkombination SDV 1000/10000 Å (Länge 65 cm), Temperatur 30 °C, THF als mobile Phase, Fließrate 1 ml/min, Probenkonzentration 10 g/l, RI-Detektor, Auswertung gegen Polypropylenglykol-Standard.

### Bestimmung des Gehalts an Propenylpolyethern:

Die Bestimmung des Gehalts an Propenylpolyethern erfolgte mit Hilfe der ¹H-NMR-Spektroskopie. Verwendet wurde ein NMR Spektrometer vom Typ Bruker Avance 400. Die Proben wurden dazu in Deuteromethanol gelöst. Der Propenylgehalt ist definiert als der Anteil an Propenylpolyethern in mol-% bezogen auf die Gesamtheit an allen in der Probe enthaltenen Polyethern.

Die quantitative Bestimmung des Propionaldehyd-Gehalts erfolgte mit Hilfe der HPLC-Methode.

### Bestimmung des Alkaligehalts in Polyethern:

Zur quantitativen Bestimmung des Gehalts an Natrium und Kalium wurden die Proben mit heißer Salpetersäure aufgeschlossen und nach der ICP-OES Methode (Optische Emissionsspektrometrie mittels induktiv gekoppeltem Plasma) vermessen.

### Bestimmung der lodzahl in Polyethern:

Die Iodzahlbestimmung erfolgte nach der Titrationsmethode von Hanus, beschrieben als Methode DGF C-V 17 a (53) der Deutschen Gesellschaft für Fettwissenschaft.

### Bestimmung der Säurezahl in Polyethern:

Die Säurezahlbestimmung wurde nach einem Titrationsverfahren in Anlehnung an die DIN EN ISO 2114 durchgeführt.

Für die erfindungsgemäßen Aufbereitungen wurden die folgenden alkalisch katalysierten Alkoxylierungsprodukte verwendet (Tabelle 1):

| Alkalischer Polyether | Chemischer Aufbau | Alkaligehalt | Katalysator | Propenylgehalt | lodzahl |
|---|---|---|---|---|---|
| AP 1 | Poly(oxypropylen)monobutylether Mw 700 g/mol, Mw/Mn 1,10 | 3100 ppm Na | Natriummethanolat | entfällt | entfällt |
| AP 2 | Poly(oxypropylen)monobutylether Mw 1800 g/mol, Mw/Mn 1,16 | 3300 ppm K | Kaliummethanolat | entfällt | entfällt |
| AP 3 | Poly(oxyethylen)-co-(oxypropylen)monobutylether Mw 1000 g/mol, Mw/Mn 1,08 50 mol-% EO, 50 mol-% PO | 1700 ppm K | Kaliummethanolat | entfällt | entfällt |
| AP 4 | Poly(oxyethylen)monoallylether Mw 400 g/mol, Mw/Mn 1,15 | 850 ppm Na | Natriummethanolat | entfällt | 64,0 g lod/100 g |
| AP 5 | Poly(oxyethylen)monoallylether Mw 600 g/mol, Mw/Mn 1,10 | 1600 ppm K | Kaliummethanolat | 0,6 mol-% | 43,0 g lod/100 g |
| AP 6 | Poly(oxyethylen)-co-(oxypropylen)monoallylether Mw 900 g/mol, Mw/Mn 1,09 70 mol-% EO, 30 mol-% PO | 1200 ppm Na | Natriummethanolat | 1,1 mol-% | 31,0 g lod/100 g |
| AP 7 | Poly(oxyethylen)-co-(oxypropylen)monoallylether Mw 4400 g/mol, Mw/Mn 1,27 50 mol-% EO, 50 mol-% PO | 1500 ppm K | Kaliummethanolat | 20,3 mol-% | 5,8 g Iod/100 g |

| | | | | | |
|---|---|---|---|---|---|
| AP 8 | Poly(oxyethylen)-co-(oxypropylen)monoallylether Mw 500 g/mol, Mw/Mn 1,14 60 mol-% EO, 40 mol-% PO | 1600 ppm Na | Natriummethanolat | 1,3 mol-% | 49,0 g lod/100 g |
| AP 9 | Poly(oxyethylen)-co-(oxypropylen)glykol Mw 2800 g/mol, Mw/Mn 1,05 55 mol-% EO, 45 mol-% PO | 4400 ppm K | Kaliumhydroxid | entfällt | entfällt |
| AP 10 | Poly(oxyethylen)-co-(oxypropylen)monoallylether Mw 1500 g/mol, Mw/Mn 1,16 10 mol-% EO, 90 mol-% PO | 2900 ppm K | Kaliummethanolat | 5,1 mol-% | 17,0 g lod/100 g |
| AP 11 | Poly(oxyethylen)-co-(oxypropylen)monoallylether Mw 4000 g/mol, Mw/Mn 1,28 50 mol-% EO, 50 mol-% PO | 2900 ppm Na | Natriummethanolat | 4,6 mol-% | 6,5 g Iod/100 g |

**Folgende Kationenaustauscher wurden eingesetzt, Herstellerangaben (Tabelle 2):**

| | | Partikelgröße | Kapazität | Wassergehalt | |
|---|---|---|---|---|---|
| Amberlyst® 15 | makroporös, SO3H-funktionell | harmonischer Mittelwert 0,60-0,85 mm | ≥1,7 eq/l, ≤4,7 eq/l | 52-57 % | erfindungsgemäß |
| Amberlite® 252H | makroporös, SO3H-funktionell | harmonischer Mittelwert 0,6-0,8 mm | >1,7 eq/l | 52-58 % | erfindungsgemäß |
| Lewatit® CNP-80 | makroporös, COOHfunktionell | 0,315-1,6 mm | >4,3 eq/l | nicht bekannt | Vergleichsbeispiel |

### Versuche zur erfindungsgemäßen Aufreinigung der alkalischen Alkoxylierungsprodukte im Rührreaktor:

In einem mit Rührer, Temperaturfühler und pH-Meter ausgestatteten temperierbaren Glasgefäß wurden gemäß Tabelle 3 jeweils 250 g eines alkalischen Polyethers (siehe Tabelle 1), Alkohol und Wasser vorgelegt und unter Rühren auf die gewünschte Temperatur gebracht. Das pH-Meter zeigte in allen Fällen einen pH-Wert von 12 bis 14. Beim Erreichen der Solltemperatur wurde die jeweilige Menge an Ionenaustauscher hinzugefügt. Mit einer Stoppuhr wurde die Zeit bis zum Erreichen eines pH-Werts von 7 gemessen.

**Tabelle 3: Aufbereitung alkalischer Alkoxylierungsprodukte (jeweils 250 g) im Rührreaktor**

| Versuch | Alkalische r Polyether | Ionenaustauscher | Lösemittel | Lösemittel [g] | Wasser [g] | Ionenaustauscher [g] | Tem p. [°C] | Zeit [min] |
|---|---|---|---|---|---|---|---|---|
| 1* | AP 2 | Amberlite® 252H | Ethanol | 250 | 10 | 25 | 45 | 35 |
| 2* | AP 2 | Amberlite® 252H | Ethanol | 125 | 10 | 25 | 45 | 45 |
| 3* | AP 2 | Amberlyst® 15 | Isopropanol | 250 | 10 | 25 | 45 | 22 |
| 4* | AP 2 | Amberlyst® 15 | Propanol | 250 | 10 | 25 | 45 | 30 |
| 5* | AP 2 | Amberlyst® 15 | Ethanol | 250 | 10 | 25 | 79 | 5 |
| 6* | AP 2 | Amberlite® 252H | Ethanol | 250 | 10 | 25 | 80 | 8 |
| 7 (nicht erfindungsgemäß) | AP 2 | Amberlite® 252H | (ohne) | 0 | 10 | 25 | 80 | 60 |
| 8* | AP 2 | Amberlite® 252H | Ethanol | 75 | 10 | 25 | 80 | 20 |
| 9 (nicht erfindungsgemäß) | AP 2 | Lewatit® CNP-80 | Ethanol | 250 | 10 | 20 | 80 | >200 |
| 10 | AP 7 | Amberlite® 252H | Methanol | 250 | 10 | 25 | 45 | 35 |
| 11 | AP 7 | Amberlyst® 15 | Ethanol | 125 | 10 | 25 | 80 | 30 |
| 12 | AP 7 | Amberlyst® 15 | Ethanol | 250 | 10 | 25 | 80 | 25 |
| 13 | AP 7 | Amberlite® 252H | Isopropanol | 250 | 10 | 25 | 45 | 56 |
| 14 | AP 7 | Amberlite® 252H | Ethanol | 75 | 10 | 25 | 80 | 20 |
| 15 | AP 5 | Amberlite® 252H | Ethanol | 250 | 10 | 10 | 45 | 58 |
| 16 | AP 5 | Amberlite® 252H | Ethanol | 125 | 10 | 10 | 45 | 45 |
| 17 | AP 5 | Amberlite® 252H | Methanol | 250 | 10 | 10 | 45 | 25 |
| 18 | AP 5 | Amberlite® 252H | Ethanol | 125 | 10 | 10 | 80 | 7 |
| 19* | AP 3 | Amberlite® 252H | Ethanol | 75 | 10 | 20 | 80 | 20 |
| 20* | AP 3 | Amberlyst® 15 | Ethanol | 125 | 10 | 20 | 80 | 17 |
| 21* | AP 3 | Amberlite® 252H | Ethanol | 250 | 10 | 20 | 80 | 10 |
| 22* | AP 3 | Amberlyst® 15 | Ethanol | 250 | 10 | 20 | 80 | 10 |
| 23 | AP 6 | Amberlite® 252H | Ethanol | 75 | 10 | 15 | 80 | 26 |
| 24 | AP 6 | Amberlyst® 15 | Ethanol | 125 | 10 | 15 | 80 | 22 |
| 25 | AP 6 | Amberlyst® 15 | Propanol | 250 | 10 | 15 | 45 | 45 |
| 26 | AP 6 | Amberlite® 252H | Ethanol | 250 | 10 | 15 | 45 | 20 |
| 27 (nicht erfindungsgemäß) | AP 6 | Amberlite® 252H | Ethanol | 250 | 0 | 15 | 45 | >240 |
| 28 | AP 8 | Amberlyst® 15 | Ethanol | 250 | 10 | 15 | 45 | 18 |
| 29* | AP 9 | Amberlite® 252H | Ethanol | 250 | 10 | 25 | 45 | 45 |
| 30* | AP 9 | Amberlite® 252H | Ethanol | 125 | 10 | 25 | 45 | 45 |
| 31* | AP 4 | Amberlite® 252H | Ethanol | 250 | 10 | 10 | 45 | 20 |
| 32* | AP 4 | Amberlite® 252H | Ethanol | 125 | 10 | 10 | 45 | 20 |
| 33 | AP 10 | Amberlyst® 15 | Ethanol | 250 | 10 | 22,5 | 45 | 23 |
| 34 | AP 10 | Amberlyst® 15 | Ethanol | 125 | 10 | 22,5 | 45 | 48 |
| 35 (nicht erfindungsgemäß) | AP 10 | Amberlyst® 15 | (ohne) | 0 | 10 | 22,5 | 45 | >210 |

| | | | | | | | | |
|---|---|---|---|---|---|---|---|---|
| *Referenzbeispiele | | | | | | | | |

Die aufbereiteten, neutralisierten Polyetherlösungen wurden durch Destillation von Alkohol und Wasser befreit und anschließend auf Alkaligehalt und Säurezahl untersucht. Alle erfindungsgemäß hergestellten Polyether hatten einen Natrium- bzw. Kaliumgehalt von <5 ppm und eine Säurezahl zwischen 0 und 0,25 mg KOH/g. Die Versuche 9, 27 und 35 mussten dagegen abgebrochen werden, da auch nach einigen Stunden pH 7 nicht erreicht wurde. Die Probe aus Versuch 7 wurde nicht untersucht, da 60 min Verweilzeit unökonomisch sind.

### Versuche zur erfindungsgemäßen Aufreinigung der alkalischen Alkoxylierungsprodukte im Festbettreaktor:

Eine mit Temperaturmessfühler und mit beheizbarem Doppelmantel ausgestattete lonenaustauschersäule von etwa 600 ml Innenvolumen wurde mit 287 g Ionenaustauscher gefüllt. Über eine regelbare Kolbenpumpe wurden je Versuch 3-5 Liter der gemäß Tabelle 4 vorbereiteten Lösungen aus alkalischem Polyether (siehe Tabelle 1) in Alkohol und Wasser über eine Versuchsdauer von einigen Stunden kontinuierlich zugespeist. Während der Versuchsdauer wurde die Innentemperatur durch Regelung der Manteltemperatur auf dem eingestellten Sollwert konstant gehalten. Über die Dosierrate der Pumpe wurde die Verweilzeit der Polyetherlösung in der Säule variiert. Der pH-Wert der am anderen Ende der Ionenaustauschersäule ausströmenden Produktlösung wurde fortwährend gemessen und zeigte in allen Fällen einen pH-Wert von <7. Die aufgereinigten Lösungen wurden in einem Behälter aufgefangen und anschließend vom jeweiligen Lösemittel befreit. Alkohol und Wasser wurden zunächst unter Normaldruck, danach unter Vakuum bei ansteigenden Temperaturen bis 120 °C abdestilliert. Es wurden klare, salzfreie neutralisierte Polyether mit einem Alkaligehalt von <5 ppm und einer Säurezahl von 0 bis 0,25 mg KOH/g erhalten.

**Tabelle 4: Aufbereitung alkalischer Alkoxylierungsprodukte im Festbettverfahren, Einsatzmengen bezogen auf 2,5 kg alkalisches Alkoxylierungsprodukt**

| Versuch | Alkalischer Polyether | Ionenaustauscher | Lösemittel | Lösemittel [g] | Wasser [g] | Einspeisung [g/min] | Temp. [°C] |
|---|---|---|---|---|---|---|---|
| A 1* | AP 1 | Amberlyst® 15 | Ethanol | 2500 | 100 | 12,9 | 47 |
| A 2* | AP 1 | Amberlite® 252H | Ethanol | 1250 | 100 | 15,3 | 80 |
| A 3* | AP 1 | Amberlite® 252H | Ethanol | 625 | 100 | 13,1 | 80 |
| A 4* | AP 2 | Amberlyst® 15 | Ethanol | 2500 | 100 | 16,0 | 45 |
| A 5* | AP 2 | Amberlite® 252H | Ethanol | 1250 | 100 | 16,0 | 78 |
| A 6* | AP 2 | Amberlite® 252H | Ethanol | 625 | 100 | 15,2 | 80 |
| A 7* | AP 3 | Amberlyst® 15 | Ethanol | 2500 | 100 | 13,2 | 45 |
| A 8* | AP 3 | Amberlyst® 15 | Isopropanol | 2500 | 100 | 13,9 | 45 |
| A 9* | AP 3 | Amberlite® 252H | Ethanol | 1250 | 100 | 16,5 | 79 |
| A 10 | AP 6 | Amberlyst® 15 | Ethanol | 2500 | 100 | 13,8 | 46 |
| A 11 | AP 6 | Amberlite® 252H | Ethanol | 1250 | 100 | 15,8 | 76 |
| A 12 | AP 7 | Amberlyst® 15 | Ethanol | 2500 | 100 | 15,1 | 45 |
| A 13 | AP 7 | Amberlite® 252H | Ethanol | 2500 | 100 | 11,8 | 78 |
| A 14 | AP 7 | Amberlite® 252H | Ethanol | 1250 | 100 | 13,4 | 78 |
| A 15 | AP 7 | Amberlite® 252H | Ethanol | 650 | 100 | 4,5 | 78 |
| A 16 | AP 8 | Amberlite® 252H | Ethanol | 2500 | 100 | 13,1 | 45 |
| A 17 | AP 8 | Amberlyst® 15 | Ethanol | 2500 | 100 | 14,2 | 45 |
| A 18 | AP 8 | Amberlite® 252H | Ethanol | 625 | 100 | 19,3 | 76 |
| A 19* | AP 4 | Amberlyst® 15 | Ethanol | 2500 | 100 | 14,4 | 46 |
| A 20* | AP 4 | Amberlite® 252H | Ethanol | 2500 | 100 | 15,6 | 79 |
| A 21* | AP 4 | Amberlite® 252H | Ethanol | 625 | 100 | 22,3 | 75 |
| A 22 | AP 5 | Amberlyst® 15 | Ethanol | 2500 | 100 | 13,4 | 45 |
| A 23 | AP 11 | Amberlite® 252H | Ethanol | 2500 | 100 | 17,7 | 80 |

| | | | | | | | |
|---|---|---|---|---|---|---|---|
| *Referenzbeispiele | | | | | | | |

Wie die nachfolgende Tabelle 5 aufzeigt, werden beim Durchleiten durch das lonenaustauscherfestbett Propenylpolyether und andere geruchsbildende Beimengungen effektiv hydrolytisch zerstört und anschließend destillativ beseitigt. Die analytischen Ergebnisse der ¹H-NMR-Spektren werden durch Iodzahlmessungen bestätigt, die auf eine Reduktion des Gehalts an Doppelbindungen im Vergleich zum jeweiligen alkalischen Ausgangspolyether hinweisen.

**Tabelle 5: Gehalte an Doppelbindungen, Propenylpolyethern und anderen Beimengungen vor und nach der erfindungsgemäßen Aufreinigung im Festbettreaktor**

| Alkalische Polyether | | | | Aufgereinigte Polyether | | | |
|---|---|---|---|---|---|---|---|
| Polyether | Iodzahl [g Iod/100 g] | Propenylgehalt [mol-%] | Propionaldehyd [ppm] | Versuch | Iodzahl [g Iod/100 g] | Propenylgehalt [mol-%] | Propionaldehyd [ppm] |
| AP 4* | 64,0 | entfällt | nicht bestimmt | A 19 | 64,0 | entfällt | nicht bestimmt |
| AP 4* | 64,0 | entfällt | nicht bestimmt | A 20 | 63,2 | entfällt | nicht bestimmt |
| AP 4* | 64,0 | entfällt | nicht bestimmt | A 21 | 63,8 | entfällt | nicht bestimmt |
| AP 5 | 43,0 | 0,6 | nicht bestimmt | A 22 | 42,8 | 0,3 | nicht bestimmt |
| AP 6 | 31,0 | 1,1 | 616 | A 11 | 30,8 | 0,4 | 6 |
| AP 7 | 5,8 | 20,3 | 2500 | A 15 | 5,3 | 3,7 | nicht bestimmt |
| AP 7 | 5,8 | 20,3 | 2500 | A 14 | 5,3 | 3,9 | nicht bestimmt |
| AP 7 | 5,8 | 20,3 | 2500 | A 12 | 4,8 | 2,4 | 370 |
| AP 8 | 49,0 | 1,3 | 940 | A 17 | 48,2 | 0,4 | nicht bestimmt |
| AP 8 | 49,0 | 1,3 | 940 | A 18 | 48,4 | 0,6 | 17 |
| AP 2* | entfällt | entfällt | 1190 | A 6 | entfällt | entfällt | <160 |
| AP 1* | entfällt | entfällt | 180 | A 3 | entfällt | entfällt | 22 |
| AP 11 | 6,5 | 4,6 | nicht bestimmt | A 23 | 6,4 | 2,1 | nicht bestimmt |

| | | | | | | | |
|---|---|---|---|---|---|---|---|
| *Referenzbeispiele | | | | | | | |

Die Ergebnisse in Tabelle 5 belegen auf eindeutige Weise die Reduktion der Propenylpolyetheranteile und der Gehalte an Propionaldehyd in den erfindungsgemäß hergestellten Polyethern

## Patentansprüche

1. Verfahren zur Aufbereitung von alkalisch katalysierten Alkoxylierungsprodukten unter Verwendung von sulfonsauren Ionenaustauschern,
umfassend
a) Bereitstellen einer Mischung, umfassend das aufzubereitende alkalisch katalysierte Alkoxylierungsprodukt, Alkohol mit 1 bis 4 Kohlenstoffatomen und Wasser,
b) Behandeln der aus Schritt a) erhaltenen Mischung mit einem sulfonsauren Kationenaustauscher bei >40 °C,
c) Abtrennung, vorzugsweise destillative Abtrennung, des Alkoxylierungsproduktes aus der in Schritt b) erhaltenen Mischung,
wobei die aufzubereitenden alkalisch katalysierten Alkoxylierungsprodukte Allyl-Polyether und darin enthaltene Propenylpolyether umfassen,
zur Entfernung von Alkalimetallresten und geruchsbildenden Beimengen aus den alkalisch katalysierten Alkoxylierungsprodukten,
**dadurch gekennzeichnet, dass** bei der Aufbereitung des alkalisch katalysierten Alkoxylierungsproduktes eine Reduzierung der Propenylgruppen erfolgt.

2. Verfahren nach Anspruch 1, **dadurch gekennzeichnet, dass** die aufzubereitenden alkalisch katalysierten Alkoxylierungsprodukte aus einem mit Alkalihydroxid und/oder Alkalialkoholat katalysierten Alkoxylierungsprozess stammen, eine Molmasse Mw bestimmt mit GPC-Methode gegen PPG-Standard von 150 g/mol bis 15 000 g/mol und eine Polydispersität von Mw/Mn von 1,04 bis 1,5 aufweisen.

3. Verfahren nach zumindest einem der Ansprüche 1 oder 2, **dadurch gekennzeichnet, dass** der verwendete Ionenaustauscher Sulfonsäuregruppen enthält, eine mittlere Partikelgröße von 500-900 µm und eine Ionenaustauschkapazität von größer oder gleich 1,5 equ./Liter (H+ Form) hat.

4. Verfahren nach zumindest einem der Ansprüche 1 bis 3, **dadurch gekennzeichnet, dass** die mit Ionenaustauscher zu behandelnde Mischung in Schritt b) zu 35 bis 95 Gew.-%, bevorzugt zu 45 bis 85 Gew.-%, besonders bevorzugt zu 50 bis 80 Gew.-% aus Alkoxylierungsprodukt, zu 4 bis 64 Gew.-%, bevorzugt 12 bis 53 Gew.-%, besonders bevorzugt 17 bis 48 Gew.-% aus Alkohol mit 1 bis 4 Kohlenstoffatomen und zu 1 bis 15 Gew.-%, bevorzugt 2 bis 10 Gew.-%, besonders bevorzugt 3 bis 8 Gew.-% aus Wasser besteht.

5. Verfahren nach zumindest einem der Ansprüche 1 bis 5, **dadurch gekennzeichnet, dass** die aufzubereitenden alkalisch katalysierten Alkoxylierungsprodukte Addukte von Alkylenoxiden, vorzugsweise aus der Gruppe von Ethylenoxid, Propylenoxid, 1-Butylenoxid, 2-Butylenoxid, Isobutylenoxid und Styroloxid, besonders bevorzugt Ethylenoxid und Propylenoxid, mit Allylalkohol sind.

6. Verfahren nach zumindest einem der Ansprüche 1 bis 5, **dadurch gekennzeichnet, dass** in Schritt b) die Mischung aus Schritt a) bei 45 °C bis 100 °C durch ein Ionenaustauscherbett geleitet wird.

7. Verfahren nach zumindest einem der Ansprüche 1 bis 6, **dadurch gekennzeichnet, dass** ein um mehr als 40 %, bevorzugt 50 % bis 95 % geringerer Gehalt an Propenylgruppen resultiert, verglichen mit dem zur Aufbereitung eingesetzten Alkoxylierungsprodukt.

## Claims

1. Method of processing of alkali-catalysed alkoxylation products using sulphonic acid-containing ion exchangers,
comprising
a) providing a mixture comprising the alkali-catalysed alkoxylation product to be processed, alcohol having 1 to 4 carbon atoms and water,
b) treating the mixture obtained from step a) with a sulphonic acid-containing cation exchanger at > 40°C,
c) removal, preferably distillative removal, of the alkoxylation product from the mixture obtained in step b),
wherein the alkali-catalysed alkoxylation products to be processed comprise allyl polyethers and propenyl polyethers present therein, for removing alkali metal residues and odour-forming additions from the alkali-catalysed alkoxylation products, **characterized in that** the processing of the alkali-catalysed alkoxylation product effects a reduction in the propenyl groups.

2. Method according to Claim 1, **characterized in that** the alkali-catalysed alkoxylation products to be processed originate from an alkali-metal-hydroxide-and/or alkali-metal-alkoxide-catalysed alkoxylation process, have a molar mass M_{W} determined by GPC against a PPG standard of 150 g/mol to 15 000 g/mol and a polydispersity Mw/Mn of 1.04 to 1.5.

3. Method according to at least one of Claims 1 or 2, **characterized in that** the ion exchanger used contains sulphonic acid groups, has an average particle size of 500-900 µm and has an ion exchange capacity of not less than 1.5 equ./litre (H⁺ Form).

4. Method according to at least one of Claims 1 to 3, **characterized in that** in step b) the mixture for treatment with the ion exchanger is composed to an extent of 35 to 95 wt%, preferably 45 to 85 wt%, particularly preferably 50 to 80 wt%, of alkoxylation product, to an extent of 4 to 64 wt%, preferably 12 to 53 wt%, particularly preferably 17 to 48 wt%, of alcohol having 1 to 4 carbon atoms and to an extent of 1 to 15 wt%, preferably 2 to 10 wt%, particularly preferably 3 to 8 wt%, of water.

5. Method according to at least one of Claims 1 to 5, **characterized in that** the alkali-catalysed alkoxylation products to be processed are adducts of alkylene oxides, preferably from the group comprising ethylene oxide, propylene oxide, 1-butylene oxide, 2-butylene oxide, isobutylene oxide and styrene oxide, particularly preferably ethylene oxide and propylene oxide, with allyl alcohol.

6. Method according to at least one of Claims 1 to 5, **characterized in that** in step b) the mixture from step a) is passed through an ion exchanger bed at 45°C to 100°C.

7. Method according to at least one of Claims 1 to 6, **characterized in that** it results in a content of propenyl groups that is more than 40%, preferably 50% to 95%, lower compared to the alkoxylation product used for processing.

## Revendications

1. Procédé pour la préparation de produits d'alcoxylation catalysée de manière alcaline avec utilisation d'échangeurs d'ions à acide sulfonique,
comprenant
a) la mise à disposition d'un mélange, comprenant le produit d'alcoxylation catalysée de manière alcaline devant être préparé, de l'alcool comportant 1 à 4 atome(s) de carbone et de l'eau,
b) traitement du mélange obtenu de l'étape a) avec un échangeur de cations à acide sulfonique à > 40 °C,
c) séparation, de préférence séparation par distillation, du produit d'alcoxylation du mélange obtenu dans l'étape b),
les produits d'alcoxylation catalysée de manière alcaline devant être préparés comprenant des allyl-polyéthers et des propénylpolyéthers contenus dans ceux-ci,
pour l'élimination de résidus de métaux alcalins et de quantités incorporées odorantes des produits d'alcoxylation catalysée de manière alcaline, **caractérisé en ce que** lors de la préparation du produit d'alcoxylation catalysée de manière alcaline, une réduction des groupes propényle a lieu.

2. Procédé selon la revendication 1, **caractérisé en ce que** les produits d'alcoxylation catalysée de manière alcaline devant être préparés proviennent d'un procédé d'alcoxylation catalysée avec un hydroxyde alcalin et/ou un alcoolate alcalin, présentent une masse molaire Mw déterminée par un procédé de CPG avec des références de PPG de 150 g/mole à 15 000 g/mole et une polydispersité de Mw/Mn de 1,04 à 1,5.

3. Procédé selon au moins l'une des revendications 1 et 2, **caractérisé en ce que** l'échangeur d'ions utilisé contient des groupes acide sulfonique, possède une grosseur moyenne de particule de 500 à 900 µm et une capacité d'échange d'ions supérieure ou égale à 1,5 éq./litre (forme H⁺).

4. Procédé selon au moins l'une des revendications 1 à 3, **caractérisé en ce que** le mélange devant être traité avec un échangeur d'ions dans l'étape b) est constitué de 35 à 95 % en poids, préférablement de 45 à 85 % en poids, particulièrement préférablement de 50 à 80 % en poids de produit d'alcoxylation, de 4 à 64 % en poids, préférablement de 12 à 53 % en poids, particulièrement préférablement de 17 à 48 % en poids d'alcool comportant 1 à 4 atome(s) de carbone et de 1 à 15 % en poids, préférablement de 2 à 10 % en poids, particulièrement préférablement de 3 à 8 % poids d'eau.

5. Procédé selon au moins l'une des revendications 1 à 5, **caractérisé en ce que** les produits d'alcoxylation catalysée de manière alcaline devant être préparés sont des adduits d'oxydes d'alkylène, de préférence du groupe de l'oxyde d'éthylène, l'oxyde de propylène, l'oxyde de 1-butylène, l'oxyde de 2-butylène, l'oxyde d'isobutylène et l'oxyde de styrène, particulièrement préférablement l'oxyde d'éthylène et l'oxyde de propylène, avec l'alcool allylique.

6. Procédé selon au moins l'une des revendications 1 à 5, **caractérisé en ce que** dans l'étape b) le mélange de l'étape a) est conduit à une température de 45 °C à 100 °C à travers un lit d'échangeur d'ions.

7. Procédé selon au moins l'une des revendications 1 à 6, **caractérisé en ce qu'**une teneur de 40 % plus faible, préférablement 50 % à 95 %, en groupes propényle en résulte, par comparaison avec le produit d'alcoxylation utilisé pour la préparation.
